# EUROPÄISCHE PATENTANMELDUNG

(11) **EP 3 249 560 A1**
(43) Veröffentlichungstag der Anmeldung: **29.11.2017**
(21) Anmeldenummer: 16171413.4
(22) Anmeldetag: 25.05.2016
(51) Int. Cl.: G06F 19/00

(54) **COMPUTERGESTÜTZTES VERFAHREN UND VORRICHTUNG ZUR AUTOMATISCHEN REZEPTABRECHNUNG**

(71) Anmelder: Pharmatechnik GmbH & Co. KG, 82319 Starnberg (DE)
(72) Erfinder: Graessner, Detlef, 82131 Gauting (DE)
(74) Vertreter: Becker Kurig Straus

(57) **Zusammenfassung**

Es wird ein computergestütztes Verfahren und eine entsprechende Vorrichtung bereitgestellt zur Übermittlung von Daten auf Verordnungen von Arznei- oder Heilmitteln bzw. von rezeptbezogenen Daten von einer Apotheke an ein Rezeptrechenzentrum, umfassend ein Scannen eines Rezepts zum Ermitteln von Bilddaten des Rezepts, ein Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird, wobei die Rezeptinformationen mindestens Informationen zur Identität eines Kostenträgers des Rezepts umfassen, ein Prüfen der Rezeptinformationen umfassend Durchführen einer Plausibilitätsprüfung anhand der Rezeptinformationen, ein Verknüpfen der rezeptbezogenen Daten mit dem Rezept in Papierform und ein elektronisches Übermitteln der rezeptbezogenen Daten an das Rezeptrechenzentrum.

## Beschreibung

Die vorliegende Erfindung betrifft ein computergestütztes Verfahren und eine Vorrichtung zur automatischen Rezeptabrechnung. Insbesondere betrifft die vorliegende Erfindung ein computergestütztes Verfahren und eine Vorrichtung, wobei die anschließende automatische Rezeptabrechnung schneller und effektiver durchgeführt werden kann.

### Stand der Technik

Rezeptabrechnungszentren, RZ, bieten Apotheken die Dienstleistung die Abrechnung von verarbeiteten Rezepten mit den Krankenkassen zu verwalten. Das RZ dient dabei als Vermittler und als Auswerter der unterschiedlichen Rezepte bzw. Arztverordnungen und stellt den Krankenkassen anschließend die benötigten Daten für die endgültige Abrechnung bereit.

Insbesondere beauftragen Apotheken die RZs zur Abrechnung von Rezepten, insbesondere der GKV-Rezepten, gegenüber den entsprechenden GKV-Kassen und den Herstellern. Dazu werden die Rezepte in dem RZ gescannt, die Rezeptdaten ermittelt und die Abrechnung mit zugehöriger Datenlieferung erstellt.

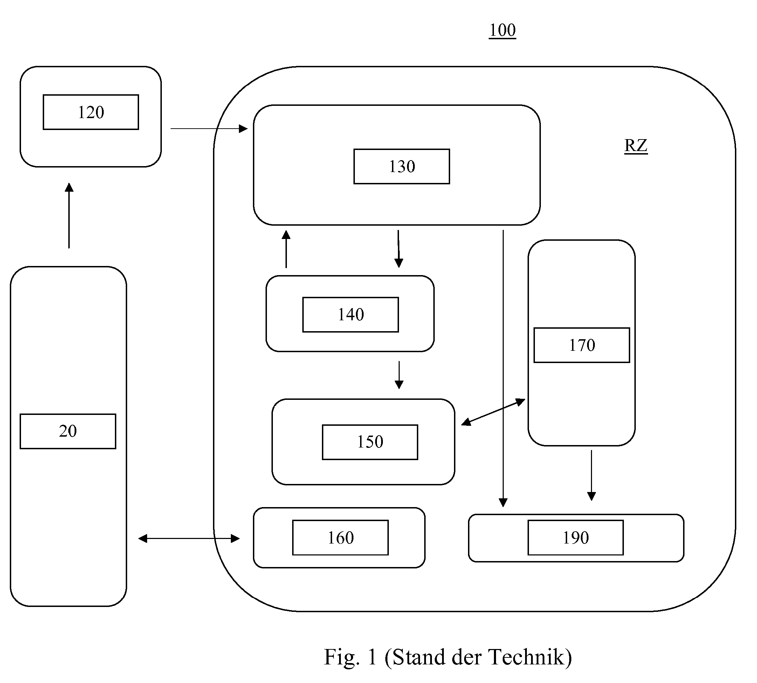

Apotheken geben Arzneimittel mit Ihrer Warenwirtschaft ab und bedrucken dabei die Rezepte. Für diesen Verarbeitungsschritt wird in vielen Systemen das Rezept vor der Abgabe mit einem Rezeptscanner eingescannt und die Arztverordnung erkannt. Die Inhalte der Rezeptbedruckung werden optional mit einer Webserviceschnittstelle (FIveRX, Rezeptvorprüfungsmodul 160 in Fig. 1) zeitnah an das jeweilige Abrechnungszentrum gesendet. Hier findet mit unmittelbarem Feedback die erste oberflächliche Rezeptkontrolle statt, d.h. im RZ analysiert eine Software die Daten der Rezeptbedruckung und teilt der Apotheke als direktes Feedback mit, ob z.B. Abrechnungsfehler, Rabattfehler oder eine mangelnde Plausibilität der herausgegebenen Rezeptzusammensetzung erkannt wurden. In diesem Fall können Fehler direkt in der Apotheke behoben werden bevor das Papierrezept zu weiteren Verarbeitung in das RZ gesendet wird.

Rezepte werden in der Regel 2x pro Monat von einem Subunternehmen in der Apotheke abgeholt und zum Rechenzentrum gebracht. Die Arbeiten in RZ gestalten sich dabei sehr aufwendig und im schlimmsten Fall treten Fehler in den Daten der Abrechnung auf, die zur vollständigen Retaxierung des Rezepts führen können.

Im Rechenzentrum müssen die Rezepte vor dem Scannen vorbereitet werden, d.h. Rezepte mit Anhängen (z.B. Hilfsmittelrezepte mit Genehmigungen, etc.) müssen von diesen separiert werden (Büroklammer entfernen, DIN A4-Zettel auffalten), Eselsohren beseitigen, Rezepte einheitlich mit Vorderseite nach vorne/hinten sortieren, usw. Für die Erfassung der Daten sind in der Regel mehrere Scanner-Durchläufe notwendig. Ein erster Durchlauf dient dazu, ein Image, also Bilddaten, vom Rezept zu generieren. Erst jetzt beginnt die Hauptarbeit im RZ: Scannerdurchläufe für eine OCR-Erkennung der Schrift auf dem Rezept, manuelle Korrektur bei Lese-/OCR-Fehlern, Ergänzung und Weiterverarbeitung der OCR-Daten für die Kostenträger (im herkömmlichen RZ werden ca. 40% der Rezepte manuell nachkorrigiert).

Im RZ erfolgt außerdem eine Erkennung von nichtbedruckten Rezepten/fehlerhaften Rezepten (keine pharmazeutische Prüfung, nur formelle regelbasierende Prüfungen). Fehlerhafte Rezepte werden entweder zurück an die Apotheke geschickt, manchmal können einzelne Fehler durch das RZ geheilt werden, was allerdings einen hohen Zeitaufwand erfordert. Weitere Scanner-Durchläufe notwendig, um alle Rezepte den Kostenträgern zuzuordnen und entsprechend zu sortieren und ggf. mit einer PIC-Nummer zu versehen (Rezepte müssen in der Regel mit aufsteigender PIC-Nummer an die jeweiligen Krankenkassen geschickt werden). Dazu bekommen sie die digitalen Daten mit dazugehörigem binarisierten Bild.

All diese Arbeiten erfordern einen hohen Aufwand an Zeit, technischen und menschlichen Ressourcen und erzeugen somit einen Großteil der entstehenden Kosten. Weitere Nachteile in den Prozeduren aktueller RZs liegen in mangelnder Transparenz und langen Wartezeiten für die Kunden. Apotheken haben individuelle Verträge mit den Rechenzentren (Gebühren sind u.a. abhängig von den monatlichen Abschlagszahlungen etc.). Rechnungen vom Rechenzentrum an die Apotheken sind oft nicht transparent, d.h. Apotheker weiß nicht, ob die Auszahlung mit dem Betrag der eingereichten Rezepte übereinstimmt.

Probleme heute:
Häufige sog. Erfassungsfehler bei der OCR-Erkennung (z.B. Zuzahlungen/Bruttobeträge falsch gelesen);
Entstehung von Dubletten, welche wiederum zu Retaxationen in den Apotheken führen;
Keinerlei Übersicht für den Apotheker, ob auch wirklich alle Rezepte im RZ erfasst wurden;
Keinerlei Möglichkeiten, das ursprüngliche Vorhandensein verloren gegangener Anhänge zu beweisen;
Hoher Zeitaufwand für die Korrektur von Erfassungsfehlern bzw. Fehler können auch übersehen werden.

Fig. 1 zeigt ein schematisches Diagramm eines Systems zur Rezeptabrechnung gemäß dem Stand der Technik. Mit Bezug auf Fig. 1 umfasst ein herkömmliches System zur Rezeptabrechnung eine Apotheke 20, eine Logistik 120, ein Belegverarbeitungsmodul 130, ein Datenerfassungsmodul 140, ein Datenablagemodul 150, eine optionale Rezeptvorprüfungsmodul 160, ein Abrechnungsmodul 170 und ein Versandmodul 190. Alle im Stand der Technik beschriebenen Module sind keine reinen Softwareapplikationen sondern können auch zum Teil oder vollständig als Abteilungen eines Systems z.B. mit rein menschlicher Arbeitskraft verstanden werden.

In dem herkömmlichen System der Fig. 1 werden in der Apotheke 20 Rezepte (bzw. allgemein Verordnungen von Arznei- oder Heilmitteln) von einem Kunden angenommen. Das Rezept wird direkt vom Apotheker ausgewertet und die ärztliche Verordnung umgesetzt. Es ist wichtig zum Verständnis der vorliegenden Erfindung, dass die Angaben auf dem Rezept durch den Arzt, z.B. die ärztliche Verordnung (z.B. Medikament X, Hersteller Y, täglich 3-mal nach dem Essen) nicht standardisiert sind. D.h. jeder Arzt kann unterschiedliche Abkürzungen in der Verordnung verwenden oder die Reihenfolge der Verordnungsparameter (im Beispiel oben Medikament, Hersteller, Dosierung) willkürlich ändern. Zudem sind auch heutzutage handschriftliche Verordnungen auf Rezepten nicht unüblich.

Es ist ebenso wichtig zu verstehen, dass die ärztliche Verordnung auf dem Rezept vom Apotheker nicht immer 1:1 umgesetzt wird. So kann es sein, dass der Apotheker den Kunden kennt und z.B. weiß dass der Kunde eine Unverträglichkeit hat oder bestimmte Vorlieben der Einnahme von Medikamenten hat. Auch kann der Apotheker Generika herausgeben, wenn z.B. dadurch Kosten für den Kunden reduziert werden. Klassische Beispiele für solche Änderungen durch den Apotheker sind "ich weiß, dass der Kunde Magenprobleme hatte, ich wähle lieber eine magenschonendere Darreichungsform" oder "der Kunde muss viele unterschiedliche Tabletten nehmen, eine farblich anders gestaltete Tablette eines anderen Herstellers hilft bei der Unterscheidung" etc.

Somit sind ärztliche Verordnung und tatsächlich verkauftes Medikament nicht immer identisch. Solche Abweichungen können aber zu Problemen bei der Abrechnung mit der zuständigen Krankenkasse führen. So ist die ärztliche Verordnung zwar nicht zwingend für die Abrechnung mit der Kasse notwendig, wird aber regelmäßig zu Kontrolle der Warenausgabe und deren Plausibilität herangezogen, wenn z.B. bestimmte Hersteller von einer Kasse bevorzugt werden, weil etwa Rabattverträge existieren. Das Auslesen der ärztlichen Verordnung im herkömmlichen RZ ist eine der größten Fehlerquellen.

Eine andere wichtige Rezeptinformation ist die Identität des Kostenträgers, also der Krankenkasse des Rezeptpatienten. Im herkömmlichen RZ wird ein großer Aufwand betrieben die Rezepte zu scannen und direkt nach Krankenkassen sortiert zu trennen. Es wird versucht Fehler in diesem Stadium der Rezeptverarbeitung durch erneute Scandurchläufe und schließlich durch manuelles Sortieren zu beheben.

Mit Bezug auf Fig. 1 werden die Rezepte im "Backoffice" der Apotheke gesammelt und über die Logistik 120 ca. zweimal monatlich durch einen Dienstleiter abgeholt und in das Rechenzentrum RZ verbracht.

In dem Belegverarbeitungsmodul 130 müssen nun eine Reihe von Arbeiten zur Vorbereitung der Rezeptprüfung durchgeführt werden. Dazu zählen Arbeitsvorbereitungen (auspacken, wiegen, ausrichten), erstes Scannen durch Hochleistungsscanner, PIC Bedruckung (Scanner), Separate Bearbeitung von Hilfsmittel- und Pflegemittelrezepten (DIN A4, in der Regel Handarbeit), ggf. Belege ziehen auf Wunsch der Apotheke, OCR (erneutes Scannen) und Rezepte kassenrein sortieren. Drei bis vier Scan-Vorgänge sind im klassischen RZ Minimum, bei Auslesefehlern erhöht sich diese Zahl deutlich.

Das Belegverarbeitungsmodul 130 arbeitet mit dem Datenerfassung- oder OCR-Modul 140 zusammen. Das Datenerfassungsmodul 140 erhält vom Belegverarbeitungsmodul 130 Imagedaten der Rezepte, um diese mit einer Texterkennungssoftware auszuwerten. Im Anschluss erhält das Belegverarbeitungsmodul 130 aus dem Datenerfassungsmodul 140 die nötigen Informationen zur Sortierung der Rezepte. Im Datenerfassungsmodul 140 werden ebenfalls die notwendigen manuellen Korrekturen der erfassten Daten durchgeführt.

Wie oben beschrieben, ist die herkömmliche Datenerfassung durch die Module 130 und 140 sehr aufwendig und geht in vielen Fällen mehrmals wechselseitig vom Belegverarbeitungsmodul 130 zum Datenerfassungsmodul 140 und wieder zurück. Bei der großen Menge an täglich verarbeiteten Rezepten führt das zu großen Verlusten von wichtigen Ressourcen. Sind Fehler auf den Rezepten auch durch Handarbeit nicht zu heilen, gehen die entsprechenden Rezepte direkt zum Postausgang 190, von wo aus sie wieder der zuständigen Apotheke 20 zugeordnet und an diese zurückgesandt werden müssen. Auch dies birgt einen erheblichen Zeit- und Verwaltungsaufwand.

Kann der Fehler in der Apotheke 20 behoben werden, kommt das Rezept erneut über die Logistik 120 im RZ an. Allerdings vergehen in der Zwischenzeit wiederrum mehrere Tage bis Wochen, wodurch die Abrechnung mit der Kasse und der Apotheke erst zu einem späteren Abrechnungszeitraum vorgenommen werden kann, was unter Umständen erhebliche finanzielle Belastungen der Apotheken mit sich bringt (einzelne Rezepte können für die Apotheke leicht einem Gegenwert von mehreren tausend Euro entsprechen).

Hat das Datenerfassungsmodul 140 schließlich alle notwendigen Daten erfasst, werden die im Datenablagemodul 150 gespeichert. Zu diesen Daten zählen Images der Rezepte, Rezeptdaten (einschließlich der Identität des Kostenträgers), Statistikdaten, Verwaltungsdaten und Artikelstammdaten.

Die Daten im Datenablagemodul 150 werden durch das Abrechnungsmodul 170 blockweise weiterverarbeitet und zusammen mit den Papierrezepten an die jeweiligen Krankenkassen versandt. Die Apotheken erhalten im Gegenzug eine Auszahlung der fälligen Erstattungen durch die Krankenkassen.

### Zusammenfassung der Erfindung

Die vorliegende Erfindung wird durch die beigefügten Ansprüche bereitgestellt. Demgemäß stellt die Erfindung in einer bevorzugten Ausführungsform ein computergestütztes Verfahren zur Übermittlung von Daten auf Verordnungen von Arznei- oder Heilmitteln bzw. von rezeptbezogenen Daten von einer Apotheke an ein Rezeptrechenzentrum bereit, das umfasst: Scannen eines Rezepts zum Ermitteln von Bilddaten des Rezepts; Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird, wobei die Rezeptinformationen mindestens Informationen zur Identität eines Kostenträgers des Rezepts umfassen; Prüfen der Rezeptinformationen durch Durchführen einer Plausibilitätsprüfung anhand der Rezeptinformationen; Verknüpfen der rezeptbezogenen Daten mit dem Rezept in Papierform; und elektronisches Übermitteln der rezeptbezogenen Daten an das Rezeptrechenzentrum.

In einer bevorzugten Ausführungsform der vorliegenden Erfindung umfassen die Rezeptinformationen weiterhin wenigstens eines, ausgewählt aus der Gruppe bestehend aus Bilddaten eines dem Rezept beigefügten Anhangs; OCR-Daten des Anhangs; ärztlichen Verordnung des Rezepts; Identität eines Arztes (z.B. Arztnummer) des Rezepts; Datum der Rezeptentgegennahme in der Apotheke und Rezeptausstellung durch den Arzt; Abgabefristen für das Rezept; Markierungen bzw. Kreuze in entsprechenden Feldern des Rezepts (z.B. "Gebührenfrei", "noctu", "Unfall", "aut idem" etc.).

In einer bevorzugten Ausführungsform der vorliegenden Erfindung erfolgt das Verknüpfen durch ein Hinzufügen eines Codes in dem Datensatz der rezeptbezogenen Daten, der eindeutig mit einem Code auf dem Rezept verknüpft ist.

In einer bevorzugten Ausführungsform der vorliegenden Erfindung liegt das Rezept in elektronischerer Form in der Apotheke vor.

In einer bevorzugten Ausführungsform der vorliegenden Erfindung beinhalten die Rezeptinformationen weiterhin zusätzliche Rezeptinformationen, die wenigstens eines der Gruppe, bestehend aus Identität der Apotheke; eine Rezeptnummer; eine Verordnungsnummer; Zahlungsinformationen; Zuzahlungsinformationen; und Informationen zur Identität des Rezeptpatienten (Versicherungsnummer) umfassen.

In einer bevorzugten Ausführungsform der vorliegenden Erfindung werden die zusätzlichen Rezeptinformationen aus einem Apotheken-Warenwirtschaftssystem, einer OCR-Erkennung der Bilddaten und/oder aus in der Apotheke aufgedruckten Daten erhalten.

In einer bevorzugten Ausführungsform der vorliegenden Erfindung umfasst das Verfahren weiterhin Ausgeben einer Warnung, falls die Plausibilitätsprüfung einen möglichen Fehler in den Rezeptinformationen bzw. den zusätzlichen Rezeptinformationen erkannt hat.

In einer weiteren bevorzugten Ausführungsform der vorliegenden Erfindung wird ein Verfahren zur Auswertung von rezeptbezogener Daten in einem Rezeptrechenzentrum bereitgestellt, umfassend elektronisches Empfangen der rezeptbezogener Daten, die gemäß einem Verfahren eines der vorstehenden Ansprüche übermittelt wurden; Empfangen eines Rezepts in Papierform, das den empfangenen rezeptbezogener Daten entspricht; Ermitteln von Bilddaten des Rezepts durch Scannen des Rezepts; Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird Abgleichen der im Rezeptrechenzentrum ermittelten rezeptbezogenen Daten des Rezepts mit den rezeptbezogenen Daten des Rezepts der elektronisch empfangen rezeptbezogener Daten; und Verwenden der elektronisch empfangen rezeptbezogener Daten für einen automatischen Rezeptabrechnungsprozess, falls das Abgleichen eine Übereinstimmung ergibt.

In einer bevorzugten Ausführungsform der vorliegenden Erfindung umfasst das weitere Verfahren weiterhin ein elektronisches Übermitteln der Bilddaten des Rezepts von der Apotheke an das Rechenzentrum und Abgleichen der Bilddaten durch Durchführen eines Bildabgleichs mittels eines vergleichenden Bildsuchalgorithmus.

Die vorliegende Erfindung stellt außerdem ein Speichermedium bereit, umfassend Programmcode, der, wenn er durch eine elektronische Vorrichtung ausgeführt wird, ein Verfahren gemäß einer der vorstehenden Ausführungsformen ausführt.

Die vorliegende Erfindung stellt außerdem ein Computerprogrammprodukt bereit, das, falls es von einer elektronischen Vorrichtung ausgeführt wird, ein Verfahren gemäß einer der Ausführungsformen ausführt.

Die vorliegende Erfindung stellt außerdem eine Vorrichtung bereit für die Durchführung eines computergestützten Verfahrens zur Übermittlung von Daten auf Verordnungen von Arznei- oder Heilmitteln bzw. von rezeptbezogenen Daten von einer Apotheke an ein Rezeptrechenzentrum gemäß einer der Ausführungsformen, umfassend einen Scanner, angepasst zum Scannen eines Rezepts zum Ermitteln von Bilddaten des Rezepts; eine Prozessoreinheit, angepasst zum Ermitteln rezeptbezogener Daten die mindestens Informationen zur Identität eines Kostenträgers des Rezepts umfassen, Prüfen der Rezeptinformationen durch Durchführen einer Plausibilitätsprüfung anhand der Rezeptinformationen und Verknüpfen der rezeptbezogenen Daten mit dem Rezept in Papierform; eine Speichereinheit; und eine Datenschnittstelle, angepasst zum elektronisches Übermitteln der rezeptbezogenen Daten an das Rezeptrechenzentrum.

In einer Ausführungsform der vorliegenden Erfindung umfasst die Vorrichtung weiterhin eine Schnittstelle zu einem Warenwirtschaftssystem der Apotheke.

In einer Ausführungsform der vorliegenden Erfindung umfasst die Vorrichtung weiterhin eine Schnittstelle zu einem Kassensystem der Apotheke zum Empfangen von Daten, die in dem Kassensystem auf das Rezept gedruckt wurden.

Die vorliegende Erfindung bietet gegenüber dem Stand der Technik die folgenden Vorteile. Bereits in der Apotheke findet eine 100%ige Rezeptkontrolle mit Hilfe einer Rezeptmanagementsoftware statt (Voraussetzung dafür ist ein Backoffice-Scanner in der Apotheke, d.h. alle bereits bedruckten Rezepte werden in der Apotheke nochmals gescannt und automatisch auf pharmazeutische und formelle Fehler geprüft). Somit können Korrekturen sofort in der Apotheke durchgeführt werden. Im Stand der Technik sind bei Feststellung irreparabler Fehler im RZ Rücksendungen der Rezepte an die Apotheke nötig. Demgegenüber bietet die Erfindung große Zeitersparnis und direkten Lernerfolg. Zudem können Rezepte auf Vollständigkeit überprüft werden (fehlt ein Rezept, so fällt dies sofort auf). Ein weiterer wichtiger Vorteil der vorliegenden Erfindung ist die verbesserte Transparenz für den Apotheker, d.h. die Apotheke weiß exakt, wieviel Erlös sie vom Rechenzentrum zu erwarten hat und die Rückmeldung ist belegbezogen.

Es gibt eine Schnittstelle zwischen der Rezeptmanagementsoftware in der Apotheke und dem RZ, d.h. alle relevanten Daten zum Rezept liegen schon bevor das physikalische Rezept im Rechenzentrum ankommt vor. Der bisherige Umweg über die OCR-Erkennung im Rechenzentrum und die damit verbundenen Fehler entfallen. Der Abgabeprozess von Hilfsmittelrezepten und Pflegehilfsmitteln wird in einer Ausführungsform der Erfindung komplett von der neuartigen Rezeptmanagementsoftware in der Apotheke unterstützt.

Die Anhänge von Hilfsmittelrezepten müssen nicht mehr mit einer Büroklammer an das Rezept geheftet werden, sondern werden in der Apotheke so markiert (d.h. verknüpft), dass diese im RZ und später auch bei der Krankenkasse dem Rezept zugeordnet werden können, was eine enorme Arbeitserleichterung darstellt. Digitale Daten zur Abrechnung von Pflegehilfsmitteln werden in einer Ausführungsform der vorliegenden Erfindung bereits in der Apotheke erzeugt. Heute erfolgt dies zu 100% manuell im Rechenzentrum und birgt einen hohen Arbeits- und Zeitaufwand. Somit werden durch die vorliegende Erfindung Arbeits- und Zeitaufwand in den RZs stark reduziert.

Weiterhin werden deutlich weniger Scanner-Durchläufe benötigt als bei den bisherigen Verfahren. Da die Daten bereits vor dem ersten Scanner-Durchlauf im RZ vorhanden sind, können die Rezepte eindeutig den Kostenträgem zugeordnet werden. Dadurch sind wir in der Lage bereits beim ersten Scanner-Durchlauf ca. 80-90% der Rezepte den Kostenträgern eindeutig zuzuordnen und diese mit einer PIC-Nummer zu versehen. Im Durchschnitt läuft ein Rezept deutlich weniger als 2x durch die Maschine (ca. 1,8). Weitere Vorteile:
- Große Transparenz für Apotheker, da alle Belege genau verfolgt werden können und ein Verlust von Dokumenten fällt sofort auf.
- Schnellere Abschlagszahlungen für den Apotheker sind möglich.
- Direkter Informationsaustausch zwischen Softwarehersteller und Rechenzentrum möglich schnelle Reaktionszeiten um Prozesse anzupassen und zu verbessern.

Die vorliegende Erfindung bedient auch ökologische Aspekte. So ist deutlich weniger Briefverkehr zwischen den Apotheken und dem RZ notwendig, weil Rezepte nicht mehr "hin und her" geschickt werden müssen, um Fehler zu beheben, die im RZ nicht geheilt werden können oder (vom Gesetz her) dürfen. Zudem sind die Laufzeiten der Scanner (in RZ und Apotheke) verkürzt, da insgesamt weniger Durchläufe benötigt werden, um alle notwendigen Daten zu erlangen, Fehler zu korrigieren und die Rezepte schließlich für die Sendung an die Kostenträger zu sortieren. Durch hohen Automatisierungs- und Optimierungsgrad wird auch ein hoher Durchsatzerreicht. Ca. 90% der Rezepte werden bei jedem Durchlauf Kassenrein sortiert.

### Kurze Beschreibung der Zeichnungen

Fig. 1 zeigt ein schematisches Diagramm eines Systems zur Rezeptabrechnung gemäß dem Stand der Technik.
Fig. 2 zeigt ein schematisches Diagramm eines computergestützten Systems zur automatischen Rezeptabrechnung mit einer Vorrichtung gemäß der vorliegenden Erfindung.
Fig. 3 ist eine detaillierte Ansicht der Vorrichtung gemäß der vorliegenden Erfindung der Ausführungsform in Fig. 2.
Fig. 4 ist ein Flussdiagramm eines Verfahrens einer Ausführungsform der vorliegenden Erfindung.

### Ausführliche Beschreibung der Ausführungsformen

Im Folgenden werden bevorzugte Ausführungsformen der vorliegenden Erfindung anhand der Zeichnungen beschrieben. Wo immer möglich und sinnvoll, wurden gleiche oder ähnliche Bezugszeichen verwendet, um gleiche oder Ähnliche Teile oder Prozesse zu bezeichnen.

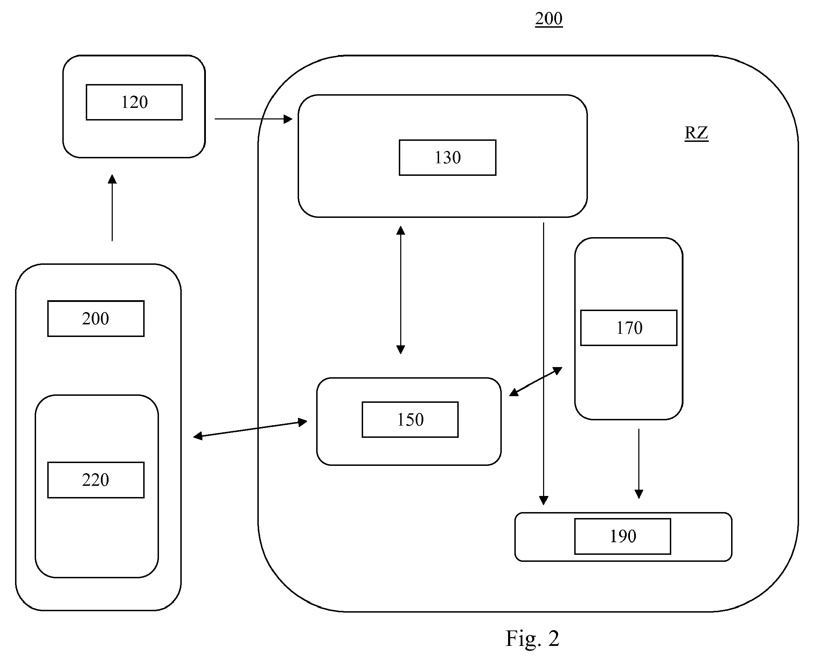

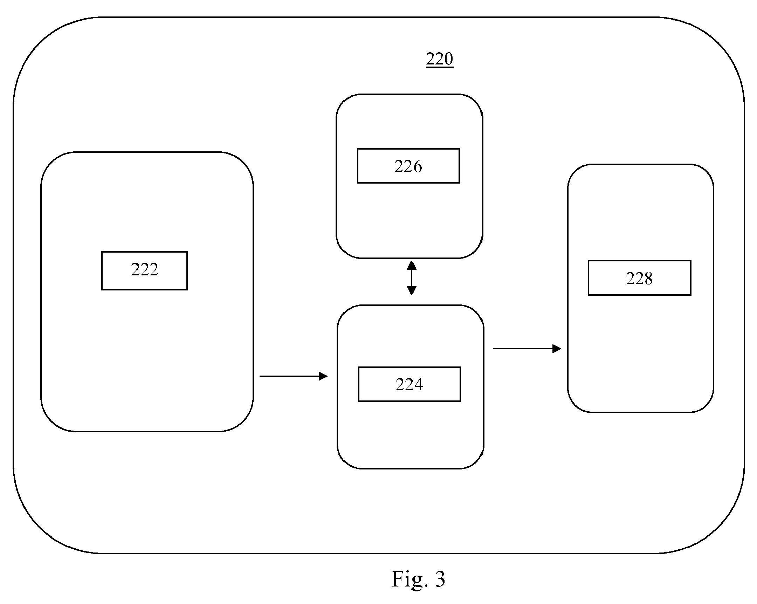

Fig. 2 zeigt ein schematisches Diagramm eines computergestützten Systems zur automatischen Rezeptabrechnung mit einer Vorrichtung einer bevorzugten Ausführungsform der vorliegenden Erfindung. Fig. 3 ist eine detaillierte Ansicht der Vorrichtung gemäß der vorliegenden Erfindung der Ausführungsform in Fig. 2.

Mit Bezug auf Fig. 2 und 3, ist das grundlegende System zur Rezeptabrechnung ähnlich dem Stand der Technik, wie er oben mit Bezug auf Fig. 1 beschreiben wurde. Alle Aspekte des Systems, die hier nicht weiter definiert werden, laufen generell auf die gleiche Art und Weise ab, wie oben mit Fig.1 beschreiben wurde.

Wie in Fig. 2 gezeigt, umfasst die Apotheke 200 im Vergleich zu der Apotheke 20 der Fig. 1 eine Vorrichtung 220, insbesondere eine Vorrichtung für die Durchführung eines computergestützten Verfahrens zur Übermittlung von rezeptbezogenen Daten von einer Apotheke an ein Rezeptrechenzentrum gemäß der vorliegenden Erfindung, im Folgenden kurz Backoffice-Vorrichtung 220 genannt. Die Backoffice-Vorrichtung 220 wird nun mit Bezug auf Fig. 3 beschrieben.

Die Backoffice-Vorrichtung 220 der Fig. 3 umfasst einen Scanner 222, eine Speichereinheit 224, eine Prozessoreinheit 226 und eine Datenschnittstelle 228. Der Scanner 222 ist angepasst zum Scannen eines Rezepts zum Ermitteln von Bilddaten des Rezepts. Der Scanner 222 übermittelt anschließend das gescannte Image des Rezepts an die Speichereinheit 224. Die Prozessoreinheit 226 kann sodann auf das Image des Rezepts und ggf. weitere rezeptbezogene Daten zugreifen. Die Prozessoreinheit 226 ist angepasst, um rezeptbezogene Daten die mindestens Informationen zur Identität eines Kostenträgers des Rezepts umfassen aus dem Image des Rezepts zu ermitteln. Dies geschieht bevorzug über eine OCR-Software aber auch andere Auswertungsmittel (Auslesen eines Codes) sind denkbar.

Die Prozessoreinheit 226 ist außerdem angepasst zum Prüfen der Rezeptinformationen umfassend Durchführen einer Plausibilitätsprüfung anhand der Rezeptinformationen und Verknüpfen der rezeptbezogenen Daten mit dem Rezept in Papierform. Die Prüfung findet, im Gegensatz zur Übermittlung der in der Apotheke aufgedruckten Daten an das Rezeptvorprüfungsmodul 160 des RZs, ausschließlich in der Apotheke, also lokal, statt. Die bringt einen Geschwindigkeitsvorteil gegenüber der externen Prüfung und lokale Gegebenheiten, z.B. individuelle Einstellungen oder Rabattverträge mit Krankenkassen, können direkt in die Prüfung einbezogen werden.

Die lokale Prüfung der Informationen bringt generell zwei entscheidende Vorteile. Zum einen können Fehler, die im Stand der Technik erst durch die Prüfung im RZ aufgedeckt worden wären, direkt in der Apotheke geheilt werden. Zum anderen wird durch die Prüfung sichergestellt, dass die erfassten Daten konsistent und plausibel sind, so dass das RZ die durch die Backoffice-Vorrichtung 220 ermittelten und geprüften Daten direkt für die Abrechnung mit den Krankenkassen verwenden kann ohne alle Schritte (siehe obige Beschreibung zur Fig. 1) selbst durchführen zu müssen. Insbesondere die Daten zur Identität des Kostenträgers (also der Krankenkasse des Rezeptpatienten) sind an dieser Stelle hervorzuheben, da diese eine wichtige Grundlage der Abrechnung mit der Krankenkasse bilden und außerdem eine sehr große Fehlerquelle bei der Erfassung der Rezeptinformationen im RZ darstellen (siehe Beschreibung der Fig.1 oben).

Wurden alle erforderlichen Daten ermittelt und die Prüfung erfolgreich abgeschlossen, werden die Daten mit dem Papierrezept verknüpft und zurück an die Speichereinheit 224 übermittelt. Die Verknüpfung erfolgt bevorzugt durch einfügen eines Codes in den Datensatz des Rezepts, der einer Kennnummer oder einem QR- bzw. Strichcode auf dem Papierrezept entspricht. Somit sind Daten und Papierrezept durch einen einfachen Scanvorgang zuzuordnen. Alternativ kann auch eine entsprechende Kennnummer bzw. ein entsprechender Code auf das Papierrezept gedruckt werden.

Von der Speichereinheit 224 werden schließlich alle Rezeptdaten elektronisch über die Datenschnittstelle 228 an das RZ übermittelt.

Mit Bezug auf Fig. 2 ergibt sich aus der Datenübermittlung von der Apotheke, genauer genommen von der Backoffice-Vorrichtung 220 an das RZ der Vorteil, dass das Datenerfassungsmodul 140 und das Prüfungsmodul 160 vollständig weggelassen werden können. Das Belegverarbeitungsmodul 130 hat in Fig. 2 nur noch die Aufgabe die eintreffenden Papierrezepte oberflächlich zu scannen. Im einfachsten Fall wird nur noch der oben erwähnte, mit den Rezeptdaten verknüpfte Code ausgelesen, die entsprechenden Daten, die im Datenablagemodul 150 von der Datenschnittstelle 228 empfangen wurden dem Rezept zugeordnet und das Papierrezept direkt kassenrein sortiert. So gut wie alle Fehlerkorrekturen und Sortierungen von Hand fallen mit dem erfindungsgemäßen System weg.

Aus dem aufwendigen Scannen, Erkennen des Kostenträgers und entsprechenden Sortieren der Rezepte im herkömmlichen RZ wird durch die vorliegende Erfindung somit ein einfaches Erkennen des Rezepts durch die eindeutige Zuordnung (z.B. Rezeptnummer, Code etc.). Die elektronisch empfangen Daten werden dem Rezept zugeordnet, wobei der Kern dieses Arbeitsschritts die Überprüfung ist, ob das Rezept in Papierform auch ordnungsgemäß versandt und empfangen wurde.

Entsprechend können die so zugeordneten Rezeptdaten mit den entsprechenden Papierrezepten direkt zum Abrechnungsmodul 170 bzw. den Postausgang 190 weitergeleitet werden ohne genaue Prüfung im RZ.

Wie oben bereits erwähnt, können somit Ressourcen im RZ geschont bzw. die verarbeiteten Rezeptzahlen erhöht werden. Sonst notwendige Spät- oder Nachtschichten können eingespart werden und die Abrechnung wird für die Apotheken transparenter. Zudem können durch wegfallen der Rezeptrücksendungen und schnellerer Vermittlung der Daten und die Krankenkassen Zahlungen an die Apotheken wesentlich schneller vorgenommen werden.

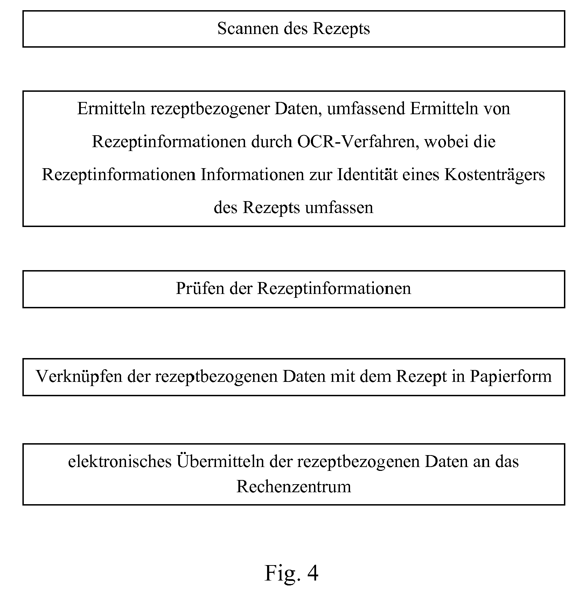

Fig. 4 ist ein Flussdiagramm eines Verfahrens einer Ausführungsform der vorliegenden Erfindung. Wie in Fig. 4 gezeigt ist, umfasst ein computergestütztes Verfahren zur Übermittlung von Daten auf Verordnungen von Arznei- oder Heilmitteln bzw. von rezeptbezogenen Daten von einer Apotheke an ein Rezeptrechenzentrum gemäß der vorliegenden Erfindung ein Scannen eines Rezepts zum Ermitteln von Bilddaten des Rezepts, ein Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird, wobei die Rezeptinformationen mindestens Informationen zur Identität des Kostenträgers des Rezepts umfassen, ein Prüfen der Rezeptinformationen umfassend Durchführen einer Plausibilitätsprüfung anhand der Rezeptinformationen, ein Verknüpfen der rezeptbezogenen Daten mit dem Rezept in Papierform und ein elektronisches Übermitteln der rezeptbezogenen Daten an das Rezeptrechenzentrum.

Im RZ werden die rezeptbezogenen Daten elektronisch empfangen. Anschließend wird das Rezept in Papierform, das den empfangenen rezeptbezogener Daten entspricht, über die Logistik empfangen. Auch im erfindungsgemäßen Verfahren werden die Bilddaten des Rezepts in Papierform durch Scannen des Rezepts ermittelt. Das Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird, reduziert sich im Idealfall im RZ auf das Auslesen eines Codes auf dem Papierrezept.

Anschließend werden die im Rezeptrechenzentrum ermittelten rezeptbezogenen Daten des Rezepts mit den verknüpften, elektronisch empfangen rezeptbezogenen Daten abgeglichen. Wie oben beschrieben reduziert sich die OCR oder Bilderkennungsverarbeitung im RZ auf die eindeutige Zuordnung vom im RZ gescannten Rezept und der elektronisch empfangen rezeptbezogenen Daten, wobei der Kern dieses Arbeitsschritts die Überprüfung ist, ob das Rezept in Papierform auch ordnungsgemäß versandt und empfangen wurde.

Bei erfolgreichem Abgleich werden Papierrezept und die entsprechenden Daten für einen automatischen Rezeptabrechnungsprozess verwendet. Ein Großteil der Arbeiten im herkömmlichen RZ werden somit eingespart und Fehler, die nicht im herkömmlichen RZ geheilt werden konnten und zu Verzögerungen und schlimmstenfalls Geldeinbußen für die Apotheken geführt haben, können durch direkte Korrektur in der Apotheke vermieden werden. Zudem ist der erfindungsgemäße Prozess für die Apotheken wesentlich transparenter.

## Patentansprüche

1. Computergestütztes Verfahren zur Übermittlung von Daten auf Verordnungen von Arznei- oder Heilmitteln bzw. von rezeptbezogenen Daten von einer Apotheke (200) an ein Rezeptrechenzentrum (RZ) zur anschließenden automatischen Rezeptabrechnung, umfassend:
Scannen eines Rezepts zum Ermitteln von Bilddaten des Rezepts;
Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird, wobei die Rezeptinformationen mindestens Informationen zur Identität eines Kostenträgers des Rezepts umfassen;
Prüfen der Rezeptinformationen, umfassend Durchführen einer Plausibilitätsprüfung anhand der Rezeptinformationen;
Verknüpfen der rezeptbezogenen Daten mit dem Rezept in Papierform; und
elektronisches Übermitteln der rezeptbezogenen Daten an das Rezeptrechenzentrum (RZ) zur automatischen Rezeptabrechnung.

2. Verfahren gemäß Anspruch 1, wobei die Rezeptinformationen weiterhin wenigstens eines umfassen, ausgewählt aus der Gruppe bestehend aus:
Bilddaten eines dem Rezept beigefügten Anhangs;
OCR-Daten des Anhangs;
Daten der ärztlichen Verordnung des Rezepts;
Identität eines Arztes des Rezepts; und
Ausstelldatum und Abgabedatum der Rezeptentgegennahme in der Apotheke.

3. Verfahren gemäß einem der vorstehenden Ansprüche, wobei das Verknüpfen durch ein Hinzufügen eines Codes in dem Datensatz der rezeptbezogenen Daten erfolgt, der eindeutig mit einem Code auf dem Rezept verknüpft ist.

4. Verfahren gemäß einem der vorstehenden Ansprüche, wobei das Rezept in elektronischerer Form in der Apotheke (200) vorliegt.

5. Verfahren gemäß einem der vorstehenden Ansprüche, wobei die Rezeptinformationen weiterhin zusätzliche Rezeptinformationen beinhalten, die wenigstens eines der Gruppe, bestehend aus
Identität der Apotheke (200);
eine Rezeptnummer;
eine Verordnungsnummer;
Zahlungsinformationen;
Zuzahlungsinformationen; und
Informationen zur Identität des Rezeptpatienten umfassen.

6. Verfahren gemäß Anspruch 5, wobei die zusätzlichen Rezeptinformationen aus einem Apotheken-Warenwirtschaftssystem, einer OCR-Erkennung der Bilddaten und/oder aus in der Apotheke (200) aufgedruckten Daten erhalten werden.

7. Computergestütztes Verfahren gemäß einem der vorstehenden Ansprüche, weiterhin umfassend:
Ausgeben einer Warnung, falls die Plausibilitätsprüfung einen möglichen Fehler in den Rezeptinformationen bzw. den zusätzlichen Rezeptinformationen erkannt hat.

8. Verfahren zur Auswertung von rezeptbezogener Daten in einem Rezeptrechenzentrum (RZ) zur anschließenden automatischen Rezeptabrechnung, umfassend:
elektronisches Empfangen der rezeptbezogener Daten, die gemäß einem Verfahren eines der vorstehenden Ansprüche übermittelt wurden;
Empfangen eines Rezepts in Papierform, das den empfangenen rezeptbezogener Daten entspricht;
Ermitteln von Bilddaten des Rezepts durch Scannen des Rezepts;
Ermitteln rezeptbezogener Daten zum Erstellen eines Datensatzes, wobei das Ermitteln der rezeptbezogenen Daten Ermitteln von Rezeptinformationen mittels eines Algorithmus zur optischen Zeichenerkennung, OCR, aus den Bilddaten des Rezepts durchgeführt wird;
Abgleichen der im Rezeptrechenzentrum ermittelten rezeptbezogenen Daten des Rezepts mit den rezeptbezogenen Daten des Rezepts der elektronisch empfangen rezeptbezogener Daten; und
Verwenden der elektronisch empfangen rezeptbezogener Daten für einen automatischen Rezeptabrechnungsprozess, falls das Abgleichen eine Übereinstimmung ergibt.

9. Verfahren gemäß Anspruch 8, weiterhin umfassend elektronisches Übermitteln der Bilddaten des Rezepts von der Apotheke (200) an das Rechenzentrum (RZ) und Abgleichen der Bilddaten durch Durchführen eines Bildabgleichs mittels eines vergleichenden Bildsuchalgorithmus.

10. Speichermedium umfassend Programmcode, der, wenn er durch eine elektronische Vorrichtung ausgeführt wird, ein Verfahren gemäß einem der vorstehenden Ansprüche ausführt.

11. Computerprogrammprodukt, das, falls es von einer elektronischen Vorrichtung ausgeführt wird, ein Verfahren gemäß einem der Ansprüche 1-9 ausführt.

12. Vorrichtung für die Durchführung eines computergestützten Verfahrens zur Übermittlung von Daten auf Verordnungen von Arznei- oder Heilmitteln bzw. von rezeptbezogenen Daten von einer Apotheke (200) an ein Rezeptrechenzentrum (RZ) gemäß einem der Ansprüche 1-7, umfassend:
einen Scanner (222), angepasst zum Scannen eines Rezepts zum Ermitteln von Bilddaten des Rezepts;
eine Prozessoreinheit (226), angepasst zum Ermitteln rezeptbezogener Daten die mindestens Informationen zur Identität eines Kostenträgers des Rezepts umfassen, Prüfen der Rezeptinformationen umfassend Durchführen einer Plausibilitätsprüfung anhand der Rezeptinformationen und Verknüpfen der rezeptbezogenen Daten mit dem Rezept in Papierform;
eine Speichereinheit (224); und
eine Datenschnittstelle (228), angepasst zum elektronisches Übermitteln der rezeptbezogenen Daten an das Rezeptrechenzentrum (RZ).

13. Vorrichtung gemäß Anspruch 12, weiterhin umfassend eine Schnittstelle zu einem Warenwirtschaftssystem der Apotheke (200).

14. Vorrichtung gemäß Anspruch 12 oder 13, weiterhin umfassend eine Schnittstelle zu einem Kassensystem der Apotheke (200) zum Empfangen von Daten, die in dem Kassensystem auf das Rezept gedruckt wurden.
